# EUROPEAN PATENT APPLICATION

(11) **EP 2 036 507 A1**
(43) Date of publication of application: **18.03.2009**
(21) Application number: 08252970.2
(22) Date of filing: 08.09.2008
(51) Int. Cl.: A61B 17/34, A61B 17/00

(54) **Composite seal and method for manufacturing**

(30) Priority: 17.09.2007 US 994048 P; 17.07.2008 US 174736
(71) Applicant: Tyco Healthcare Group LP, North Haven, CT 06473 (US)
(72) Inventor: Berry, Danny, Hamden, CT 06514 (US); Bettuchi, Michael, Middletown, CT 06457 (US); Gresham, Richard D., Guilford, CT 06437 (US); Izzo, Steven L., Naugatuck, CT 06770 (US)
(74) Representative: Alcock, David

(57) **Abstract**

The present disclosure provides a composite surgical seal for use in a surgical access device which defines an access channel through it and includes a seal member configured and dimensioned to form a seal with a housing interior wall of a surgical access device. The seal member includes a layer defining an orifice therethrough and a fabric layer substantially encapsulating the resilient layer such that a surface of the resilient layer which defines the orifice is covered by the fabric layer. The access channel is configured and dimensioned such that insertion of a surgical instrument into the access channel causes the seal member to form a substantial sealing relation with the surgical instrument inserted therethrough. A method of forming a composite surgical seal in accordance with the present disclosure is also provided herewith.

## Description

### CROSS REFERENCE TO RELATED APPLICATION

The present application claims the benefit of and priority to U.S. Provisional Application Serial No. 60/994,048 filed on September 17, 2007, the entire contents of which are incorporated herein by reference.

### BACKGROUND

### TECHNICAL FIELD

The present disclosure relates to surgical devices and, more particularly, relates to a seal assembly for use with a surgical access device during a minimally invasive surgical procedure, for example, in both laparoscopic and endoscopic procedures.

### DESCRIPTION OF RELATED ART

Minimally invasive surgical procedures avoid open invasive surgery in favor of closed or local surgery with less trauma. These procedures involve use of laparoscopic devices and remote-control manipulation of instruments with indirect observation of the surgical field through an endoscope or similar device, and are carried out through the skin or through a body cavity or anatomical opening. Laparoscopic and endoscopic procedures generally require that any instrumentation inserted into the body be sealed, i.e. provisions must be made to ensure that gases do not enter or exit the body through the incision as, for example, in surgical procedures in which the surgical region is insufflated. These procedures typically employ surgical instruments which are introduced into the body through a cannula. The cannula has a seal assembly associated therewith and provides a substantially fluid tight seal about the instrument to preserve the integrity of the established air or gas within the surgical region.

Minimally invasive procedures have several advantages over traditional open surgery, including less patient trauma, reduced recovery time, reduced potential for infection, etc. However, minimally invasive surgery, such as laparoscopy, has several disadvantages. In particular, the frictional forces exerted on surgical instruments inserted through it, has proved to be difficult in procedures requiring extensive manipulation of the long narrow endoscopic instruments within a remote site because of the restricted mobility. In addition, known seal devices are deficient in resilience and in rigidity for affixing the seal within a cannula or trocar housing.

### SUMMARY

A surgical access device is provided which includes a housing having an interior wall defining a longitudinal axis and having at least one aperture configured and dimensioned to permit passage of a surgical instrument through the aperture. The surgical access device includes a seal member supported in the housing and defining an access channel through the seal member. The seal member includes a resilient layer forming a seal with the housing interior wall and defining an orifice through it. The seal member also includes a fabric layer substantially encapsulating the resilient layer such that a surface of the resilient layer which defines the orifice is covered by the fabric layer. The access channel is configured and dimensioned such that insertion of a surgical instrument into the access channel causes the seal member to form a substantial sealing relation with the surgical instrument when it is inserted therethrough.

A composite surgical seal is provided for use in a surgical access device defining an access channel through the seal, and includes a seal member configured and dimensioned to form a seal with a housing interior wall of a surgical access device. The seal member includes a resilient layer defining an orifice therethrough and a fabric layer substantially encapsulating the resilient layer such that a surface of the resilient layer which defines the orifice is covered by the fabric layer. The access channel is configured and dimensioned such that insertion of a surgical instrument into the access channel causes the seal member to form a substantial sealing relation with the surgical instrument inserted therethrough.

A method of forming a composite seal assembly for use in a surgical access device is also provided whereby the steps include initially providing first and second fabric ring assemblies each including a rigid ring having a fabric layer secured to it. The first and second fabric ring assemblies are then positioned in opposing relation to each other such that a gap is created between them preceding approximation of opposing central portions of each fabric layer. Subsequently, a gel material is introduced between the first and second fabric layers to fill the gap formed between the first and second fabric ring assemblies to form the seal assembly. An orifice is then formed through a central portion of the seal assembly such that the fabric layer of one or both of the fabric ring assemblies covers the surface of the orifice.

The seal member may also include a rigid ring layer attached to an outer circumference of at least one of a proximal end or distal end of the seal member and is adapted for mounting to the housing.

### BRIEF DESCRIPTION OF THE DRAWINGS

Various embodiments of the present disclosure will be described hereinbelow with reference to the figures wherein:
**FIGS. 1** and **2** are perspective views of an access assembly and a seal assembly in accordance with the principles of the present disclosure;
**FIG. 3** is a perspective view with parts separated of the access and seal assemblies of **FIG. 1****;**
**FIG. 4** is a partial side cross-sectional view of the access and seal assemblies;
**FIG. 5** is a perspective view illustrating a seal assembly in accordance with the present disclosure;
**FIG. 6A** is an enlarged side cross-sectional view of an embodiment of the seal assembly of **FIG. 1****;**
**FIG. 6B** is an enlarged view of the indicated area of detail of the seal assembly of **FIG. 6A****;**
**FIG. 7A** is a top plan view of the seal assembly;
**FIG. 7B** is a side cross sectional view of the seal assembly of **FIG. 7A** taken along section line **A-A;** and
**FIG. 8** is a flow chart illustrating the steps of a method for forming a seal assembly in accordance with the present disclosure.

### DETAILED DESCRIPTION

The seal assembly of the present disclosure, either alone or in combination with a seal system internal to a cannula assembly, provides a substantial seal between a body cavity of a patient and the outside atmosphere before, during and after insertion of an object through the cannula assembly. Moreover, the seal assembly of the present disclosure is capable of accommodating objects of varying diameters, e.g., instruments from about **4.5** mm to about **15** mm, while maintaining a fluid tight interface about the instrumentation adapted for insertion through a trocar and/or cannula assembly to prevent gas and/or fluid leakage so as to preserve the atmospheric integrity of a surgical procedure. The flexibility of the present seal assembly greatly facilitates endoscopic and/or laparoscopic surgery where a variety of instruments having differing diameters are often needed during a single surgical procedure. Specifically, the surgical device includes a seal assembly which facilitates lateral and/or angular manipulation of the surgical instrument while also maintaining a seal about the instrument. The seal assembly is further adapted to substantially close in the absence of a surgical instrument to maintain the integrity of the insufflated peritoneal cavity.

The surgical seal assembly of the present disclosure is additionally adapted to decrease the frictional forces exerted on surgical instruments inserted through it which has proven to be difficult in procedures requiring extensive manipulation of the long narrow endoscopic instruments within a remote site because of the restricted mobility.

Moreover, the manufacturing of a durable seal assembly for use with a surgical access device has proven to be expensive and lacking effectiveness with maintaining good seal properties. The present disclosure provides for a more efficient and cost effective way of manufacturing a seal assembly to provide good sealing and durable properties. Specifically, the manufacturing of the seal assembly of the present disclosure provides for effective sealing properties during on and off axis motion while reducing the frictional forces of the surgical instruments lodged therethrough.

Examples of surgical instrumentation include clip appliers, graspers, dissectors, retractors, staplers, laser probes, photographic devices, endoscopes and laparoscopes, tubes, and the like. Such instruments will be collectively referred to herein as "instruments" or "instrumentation".

The seal assembly may also be adapted dimensionally to receive and form a seal about a physician's arm or hand during a hand-assisted laparoscopic procedure. In this application, the seal assembly is a component of an access member which is introduced within the body to provide access to underlying tissue in, e.g., the abdominal cavity.

Moreover, the seal assembly may be readily incorporated into an access device, such as a conventional trocar device or cannula housing to provide the device with zero-closure and/or sealing around an instrument or other object.

The subject matter of this disclosure generally relates to the subject matter of commonly assigned U.S. provisional application entitled SURGICAL PORTAL WITH GEL AND FABRIC SEAL ASSEMBLY filed under Express Mail Certificate No. EM 075410446 US on September 17, 2007, Attorney Docket No. H-US-00806 (203-5424), the entire contents of which are incorporated herein by reference.

In the following discussion, the term "proximal" or "trailing" will refer to the portion of the surgical device nearest to the clinician during operation while the term "distal" or "leading" will refer to that portion of the portal apparatus most remote to the clinician.

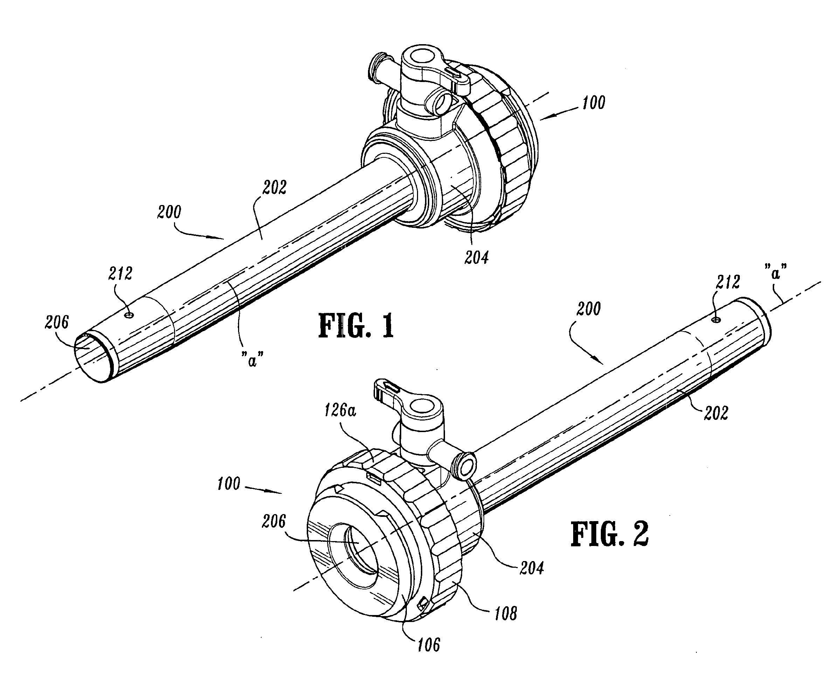

Referring now to the drawings, in which like reference numerals identify identical or substantially similar parts throughout the several views, **FIGS. 1-2** illustrate one embodiment of a seal assembly, i.e. seal assembly **100** of the present disclosure mounted to an access device **200** such as cannula or trocar assembly. Cannula assembly **200** may be any conventional cannula suitable for the intended purpose of accessing a body cavity and typically defines a passageway permitting introduction of instruments therethrough. Cannula assembly **200** is particularly adapted for use in laparoscopic surgery where the peritoneal cavity is insufflated with a suitable gas, e.g., CO₂, to raise the cavity wall from the internal organs therein. Cannula assembly **200** is typically used with an obturator assembly'(not shown) which may be blunt, a non-bladed, or a sharp pointed instrument positionable within the passageway of the cannula assembly **200.** The obturator assembly is utilized to penetrate the abdominal wall or introduce the cannula assembly **200** through the abdominal wall, and then subsequently is removed from the access device to permit introduction of the surgical instrumentation utilized to perform the procedure through the passageway.

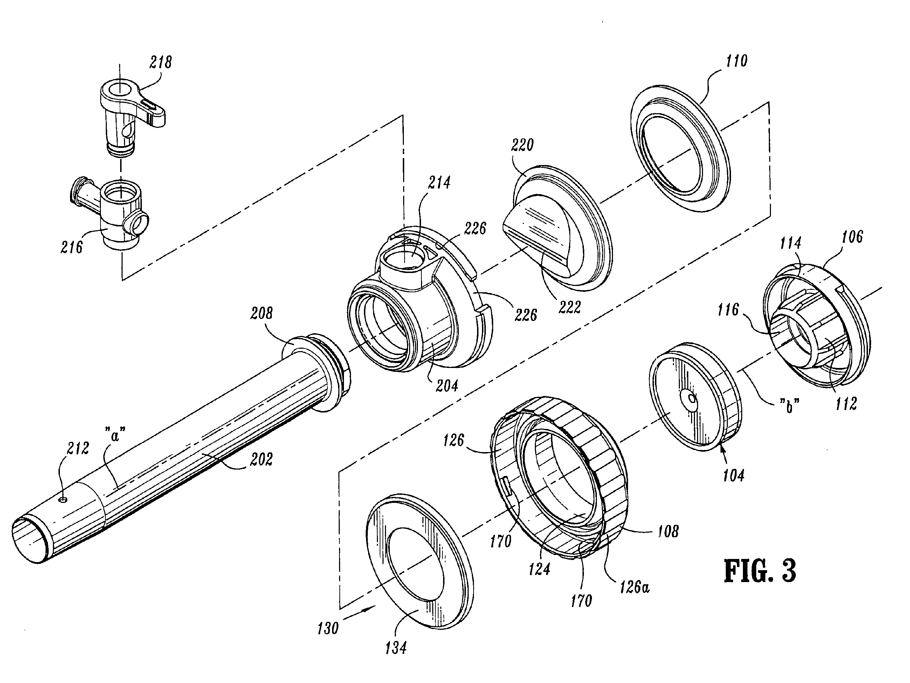

With reference to **FIGS. 1-4**, cannula assembly **200** includes cannula sleeve **202** and cannula housing **204** mounted to an end of the sleeve **202.** Any means for mounting cannula sleeve **202** to cannula housing **204** are envisioned including threaded arrangements, bayonet coupling, snap-fit arrangements, adhesives, etc. Cannula sleeve **202** and cannula housing **204** may be integrally formed. Cannula sleeve **202** defines a longitudinal axis "a" extending along the length of sleeve **202.** Sleeve **202** further defines an internal longitudinal passage **206** dimensioned to permit passage of surgical instrumentation. Sleeve **202** defines collar **208** which is mounted to cannula housing **202** and an inner tapered wall **210** adjacent the collar **208.** The sloped configuration of tapered wall **210** may assist in guiding the inserted instrument into longitudinal passage **206.** Adjacent the distal end of cannula sleeve **202** is aperture **212** which extends through the wall of the sleeve **202.** Aperture **212** permits passage of insufflation gases through cannula sleeve **202** during the surgical procedure. Sleeve **202** may be formed of stainless steel or other rigid materials such as a polymeric material or the like. Sleeve **202** may be clear or opaque. The diameter of sleeve **202** may vary, but, typically ranges from about **10** mm to about **15** mm for use with the seal assembly **100** of the present disclosure.

Cannula housing **204** includes port opening **214** and luer fitting **216** positioned within the port opening **214.** Luer fitting **216** is adapted for connection to a supply of insufflation gaseous is conventional in the art and incorporates valve **218** to selectively open and close the passage of the luer fitting **216.** Cannula housing **204** further includes duckbill or zero closure valve **220** which tapers distally and inwardly to a sealed configuration. Closure valve **220** defines slit **222** which opens to permit passage of the surgical instrumentation and closes in the absence of the instrumentation. Closure valve **220** is preferably adapted to close upon exposure to the forces exerted by the insufflation gases in the internal cavity. Other zero closure valves are also contemplated including single or multiple slit valve arrangements, trumpet valves, flapper valves, etc. Closure valve **220** rests upon internal shelf **224 of** cannula housing **204** when assembled.

Cannula housing **204** includes at least one locking recess **226** preferably two recesses arranged in diametrical opposed relation. Locking recesses **226** serve to releasably secure seal assembly **100** to cannula assembly **200.**

With continued reference to **FIGS. 1-4****,** seal assembly **100** will be discussed in detail. Seal assembly **100** may be a separate component from cannula assembly **200** and, accordingly, adapted for releasable connection to the cannula assembly **200.** Alternatively, seal assembly **100** may be incorporated as part of cannula assembly **200.** Seal assembly **100** includes a seal housing, generally identified as reference numeral **102,** and seal member **104** which is disposed within the seal housing **102.** Seal housing **102** houses the sealing components of the assembly and defines the outer valve or seal body of the seal assembly **100.** Seal housing **102** defines central seal housing axis "b" which is preferably parallel to the axis "a" of cannula sleeve **202** and, more specifically, coincident with the axis "a" of the cannula sleeve **202.** Seal housing **102** incorporates three housing components, namely, first, second and third housing components **106, 108, 110,** respectively, which, when assembled together, form the seal housing **102.** Assembly of housing components **106, 108, 110** may be affected by any of the aforementioned connection means discussed with respect to cannula housing **204.**

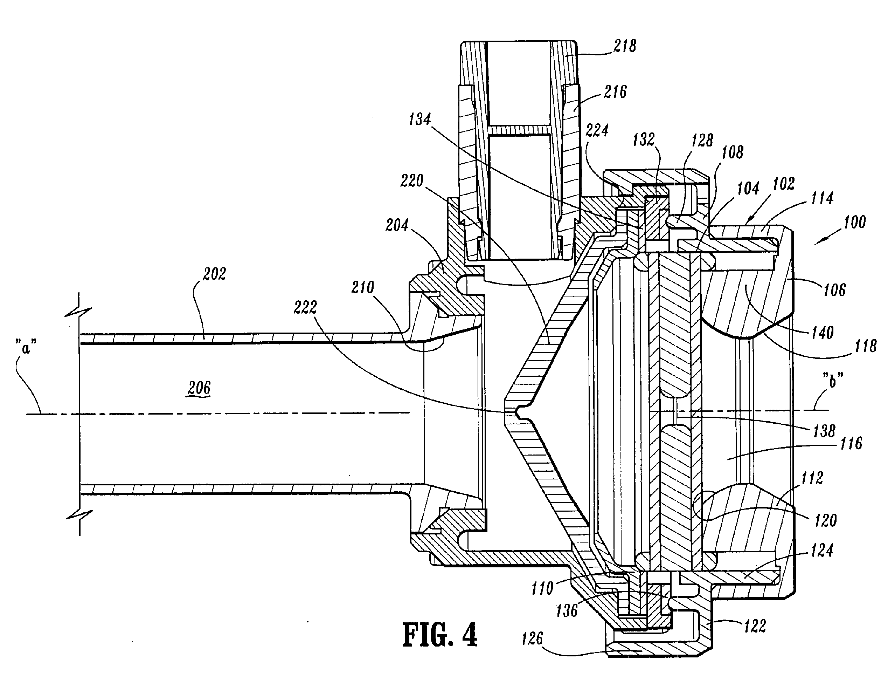

First housing component **106** defines inner guide wall **112** and outer wall **114** disposed radially outwardly of the inner guide wall **112.** Inner guide wall **112** defines central passage **116** which is dimensioned to receive a surgical instrument and laterally confine the instrument within seal housing **102.** As best shown in **FIG. 4****,** inner guide wall **112** defines sloped or tapered portion **118** adjacent its proximal end. Sloped portion **118** is obliquely arranged relative to seal housing axis "b" and extends radially inwardly relative to the seal housing axis "b" in the distal direction. Sloped portion **118** assists in guiding the inserted instrument into central passage **116,** particularly, when the instrument is non-aligned or off-axis relative to the seal housing axis "b", or introduced at an angle relative to the seal housing axis "b". Sloped portion **118** provides more flexibility to the surgeon by removing the necessity that the instrument be substantially aligned with the seal housing axis "b" upon insertion. Inner guide wall **112** is generally cylindrical in configuration and terminates in a distal arcuate or rounded surface **120.**

Second housing component **108** includes transverse wall **122,** inner cylindrical wall **124** depending in a proximal direction outwardly from the transverse wall **120** and outer wall **126** depending in a distal direction outwardly from the transverse wall **120.** Inner cylindrical wall **124** is dimensioned to mate with outer wall **114** of first housing component **106,** e.g., in a manner to be positioned within the interior of the outer wall **114** in frictional relation therewith. In the alternative, outer wall **114** of first housing component **106** may be adhered to inner cylindrical wall **124** of second housing component **108.** Outer wall **126** defines scalloped outer surface **126a** which is dimensioned for gripping engagement by the user.

In one embodiment, seal member **104** is mounted to proximal housing component **106** through, e.g., conventional means, such as by adhering the seal member **104** to the housing component **106** or molding the seal member **104** in the housing component **106.** Seal member **104** may be fabricated from an elastomer such as a soft urethane gel, silicone gel, thermoplastic elastomer (TPE) or the like and preferably has compressible characteristics to permit the seal to receive objects having a variety of sizes, to conform and form a seal about the outer surface of the inserted object, and to close upon removal of the object. Seal member **104** may be capable of accommodating instruments of varying diameters, e.g. from about **5** mm to about **12** mm, while providing a fluid tight seal with the outer diameter of each instrument. Seal member **104** may include a central orifice **138** advantageously dimensioned to permit reception and passage of a surgical instrument. In particular, orifice **138** expands upon insertion of the surgical instrument to permit passage of the surgical instrument whereby the surface portions defining orifice **138** engage the instrument in sealed relation therewith. The orifice **138** is further adapted to assume a substantially reduced dimension upon removal of the instrument. In this position, the seal member **104** restricts the egress of gaseous matter through seal housing **102.** Orifice **138** may have shapes other than that of a circular cross section opening, such as "t"-shaped, "x" shaped, helical, etc.

The use of the seal assembly **100** and cannula assembly **200** in connection with introduction of a surgical instrument will be discussed. Seal assembly **100** is mounted to cannula assembly **200** and cannula assembly **200** is introduced into an insufflated abdominal cavity. An object, e.g., an instrument, is inserted into seal assembly **100** through orifice **138** whereby the portions defining the orifice **138** stretch to accommodate the instrument diameter, as necessary. The instrument is distally passed through the valve **220** in sealed relation therewith and into the body cavity to perform the desired procedure. The instrument is removed and the orifice **138** of the seal member **104** returns to a reduced diameter configuration to assist in maintaining the integrity of the established pneumoperitoneum. Other instruments may be introduced through the seal assembly **100** and access device to perform further operative techniques as desired.

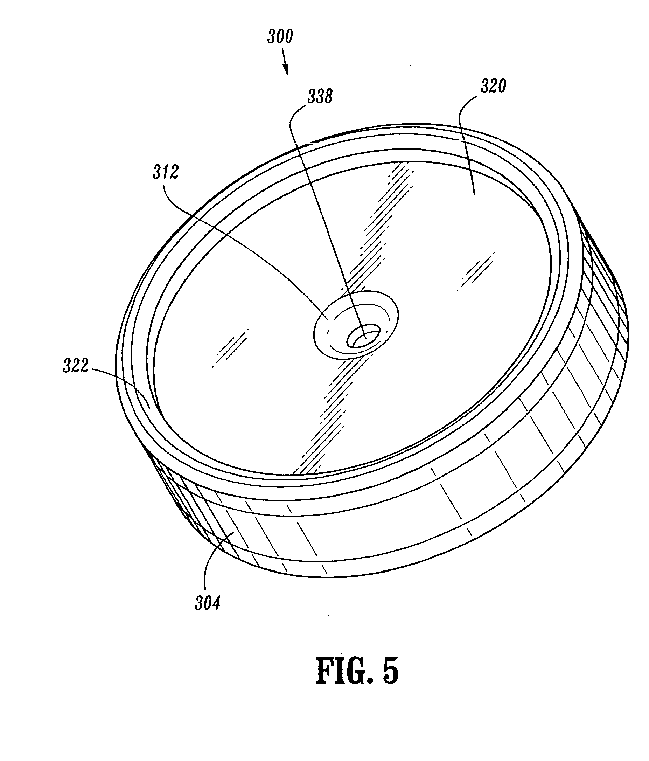

**FIG. 5** illustrates a composite seal member **300** as an exemplary embodiment of a seal member in accordance with the present disclosure. Seal member 300 is configured and dimensioned to be supported within the housing of the surgical access device, e.g., between suitable surfaces of seal housing **102,** and to define an access channel **312** therein. Seal member **300** includes a gel layer **304** which forms a seal with the housing interior wall and defining an orifice **338** therethrough. A fabric layer **320** sits above and below gel layer **304** such that a surface **320** of the gel layer **304** which defines orifice **338** is covered by fabric layer **320.** Access channel **312** is configured and dimensioned such that insertion of a surgical instrument into access channel **312** causes seal member **300** to form a substantial sealing relation with the surgical instrument inserted therethrough.

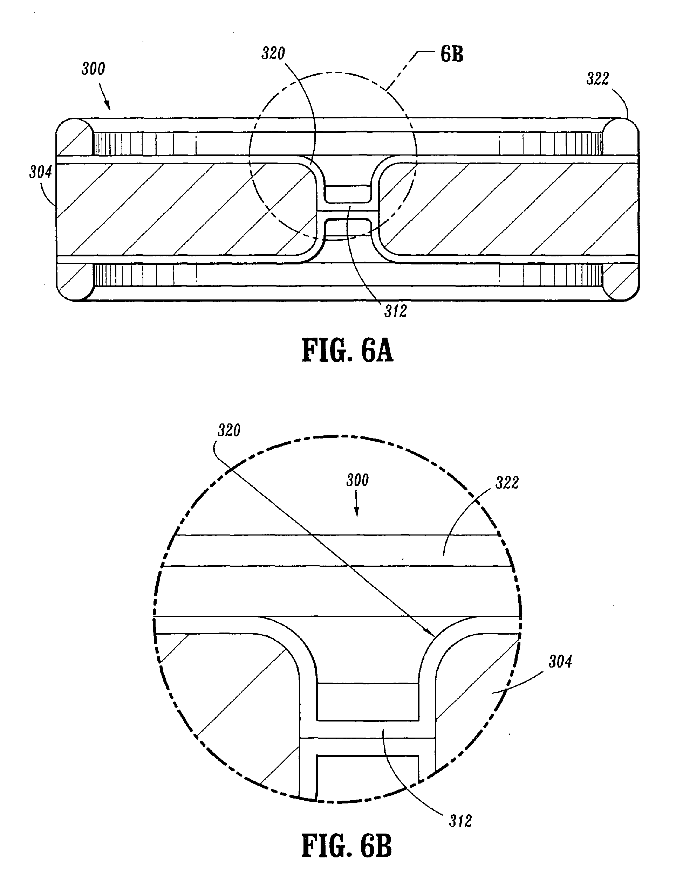

Referring now to **FIGS. 6A** and **6B**, composite seal member **300** includes a seal assembly fabricated from a first generally soft gel layer **304** and an elastic fabric layer **320** which substantially encapsulates gel layer **304.** **FIG. 6B** is an exploded view of the central portion of seal member **300** illustrating fabric layer **320** substantially encapsulating gel layer **304** and wherein fabric layer **320** also covers the surface of gel layer **304** which defines orifice **338.**

In one embodiment, gel layer **304** may be any suitable material identified hereinabove in connection with the embodiment of **FIGS. 1-4**, including, for example, a thermoplastic elastomer (TPE) or other flexible lubricous material, urethane gel, a silicon gel, alginates, gum Arabic, polymer hydrogels or a polymeric thereof, or any combination of these materials. Fabric layer **320** may include a SPANDEX® material containing **20**% LYCRA® available from Milliken. Alternatively, fabric layer **320** may be disposed on just one of either the proximally facing surface or the distally facing surface of seal member **300,** as desired (not shown).

Fabric layer **320** provides a degree of rigidity to gel layer **304** and may desirably assist in maintaining gel layer **304** in its disc-shaped configuration. Moreover, the combination of fabric layer **320** and gel layer **304** defines a seal having enhanced adaptability to a variety of different diameter objects, e.g., instruments, and which maintains a seal upon offset manipulation of the object. Fabric layer **320** also serves as a secondary seal supplemental to the sealing functions of gel layer **304.**

In still further embodiments, the seal member may include a coating to reduce frictional forces on the surgical instrument. The coating may be, e.g., an amorphous diamond coating, ion implantation coating, silicon or hydrogel coating, TEFLON® etc. and allows for more efficient manipulation of instrumentation through the access channel of a cannula or trocar.

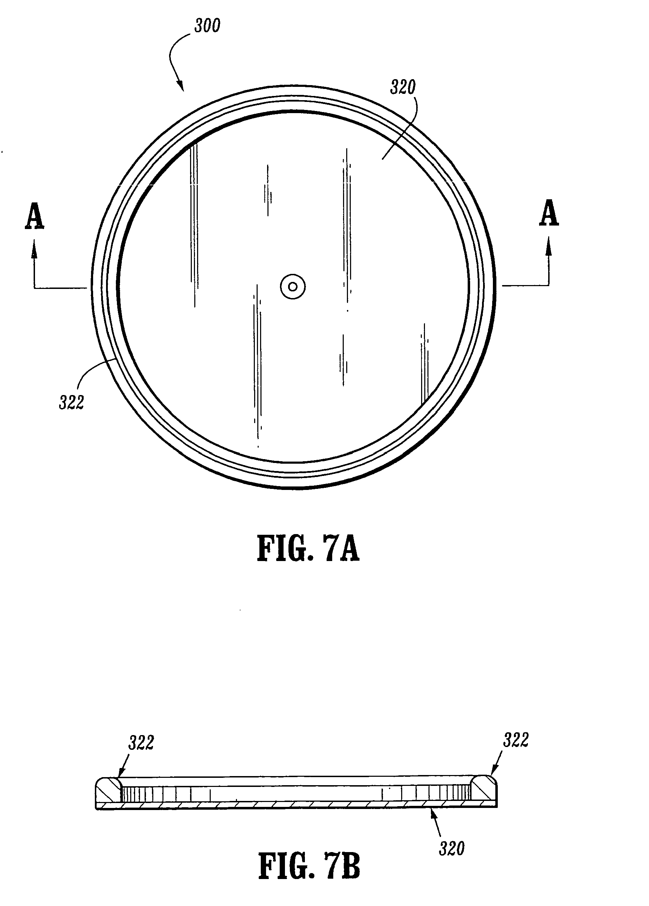

Referring now to **FIGS. 7A** and **7B****,** in one embodiment, seal member **300** illustrates a rigid ring layer **322** attached to an outer circumference of at least one of a proximal end or distal end of seal member **300.** Ring layer **322** is adapted for mounting to the housing of seal member **300.** Rigid ring layer **322** is made from a material such as nylon or any other rigid thermoplastic, e.g., polypropylene, polyethylene, polycarbonate, etc., and provides for a method for fixing the seal within a housing of a surgical access device.

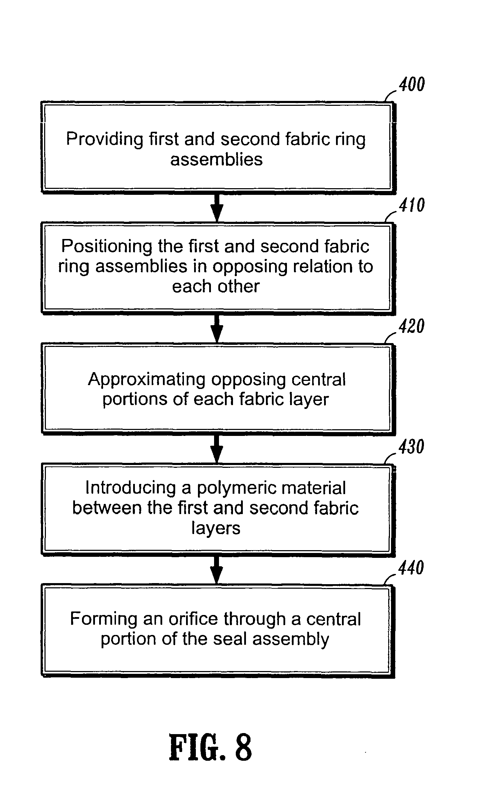

In one embodiment of a method of forming a composite seal assembly for use in a surgical access device as illustrated in the flow chart of **FIG. 8**, includes the step of initially providing first and second fabric ring assemblies each including a rigid ring having a fabric layer secured thereto (Block **400**). The first and second fabric ring assemblies are then positioned in opposing relation to each other such that a gap is created therebetween (Block **410**). Thereafter, opposing central portions of each fabric layer are approximated (Block **420**) prior to introducing a gel material between the first and second fabric layers to fill the gap formed between the first and second fabric ring assemblies which forms the seal assembly (Block **430**). An orifice is then formed through a central portion of the seal assembly such that the fabric layer of one or both of the fabric ring assemblies covers the surface of the orifice (Block **440**).

In more detail, in some embodiments, a composite seal assembly for use in a surgical access device is formed by securing a first fabric ring assembly to a first fabric layer and a second fabric ring assembly to a second fabric layer via overmolding the fabric ring assemblies onto the fabric layers. Additionally, excess fabric on the orifice of the seal member is removed. The first and second fabric ring assemblies are then positioned in opposing relation to each other such that a gap is created therebetween via pressing the fabric ring assemblies into recesses of a mold.

The opposing central portions of each fabric layer are then pinched together with mating core pins creating an access channel for instruments to pass therebetween having good sealing properties. A thin layer of fabric at the central portion of the seal assembly is thereafter removed to form the orifice **324** which defines the access channel **312** such that the orifice accepts surgical instruments therethrough. In further embodiments, a coating is applied to the seal member to reduce frictional force with the surgical instruments. The coating may be, e.g., an amorphous diamond coating, ion implantation coating, silicon or hydrogel coating , TEFLON® coating etc., and allows for more efficient manipulation of instrumentation through the access channel of a cannula or trocar.

While the above description contains many specifics, these specifics should not be construed as limitations on the scope of the disclosure, but merely as exemplifications of embodiments thereof. Those skilled in the art will envision many other possibilities within the scope and spirit of the disclosure as defined by the claims appended hereto.

## Claims

1. A surgical access device, comprising:
a housing having an interior wall defining a longitudinal axis and having at least one aperture configured and dimensioned to permit passage of a surgical instrument therethrough,
a seal member supported in the housing and defining an access channel therein, the seal member including:
a resilient layer forming a seal with the housing interior wall and defining an orifice therethrough; and
a fabric layer substantially encapsulating the resilient layer such that a surface of the resilient layer which defines the orifice is covered by the fabric layer;
wherein the access channel is configured and dimensioned such that insertion of a surgical instrument into the access channel causes the seal member to form a substantial sealing relation with the surgical instrument inserted therethrough.

2. The surgical device according to claim 1, wherein the seal member further comprises a rigid ring layer attached to an outer circumference of at least one of a proximal end or distal end of the seal member.

3. The surgical access device according to claim 2, wherein the rigid ring layer is adapted for mounting to the housing.

4. The surgical access device according to claim 1, 2 or 3, wherein the resilient layer comprises a thermoplastic elastomer.

5. The surgical access device according to claim 4, wherein the thermoplastic elastomer comprises a gel.

6. The surgical access device according to any one of the preceding claims, wherein the fabric material comprises spandex.

7. The surgical access device according to any one of the preceding claims, wherein the surgical access device is a cannula.

8. The surgical access device according to claim 2, wherein the rigid ring layer includes a material selected from the group consisting of nylon, polypropylene, polyethylene or polycarbonate.

9. A method of forming a composite seal assembly for use in a surgical access device, comprising the steps of:
providing first and second fabric ring assemblies each including a rigid ring having a fabric layer secured thereto;
positioning the first and second fabric ring assemblies in opposing relation to each other such that a gap is created therebetween;
approximating opposing central portions of each fabric layer; introducing a gel material between the first and second fabric layers to fill the gap formed between the first and second fabric ring assemblies to form the seal assembly; and
forming an orifice through a central portion of the seal assembly.

10. The method of forming a composite seal assembly for use in a surgical access device according to claim 9, wherein the step of forming an orifice through a central portion of the seal assembly further includes covering the surface of the orifice with the fabric layer of one or both of the fabric ring assemblies.

11. The method of forming a composite seal assembly for use in a surgical access device according to claim 9 or 10, wherein the providing step includes securing a first fabric ring assembly to a first fabric layer and a second fabric ring assembly with a second fabric layer.

12. The method of forming a composite seal assembly for use in a surgical access device according to claim 9, 10 or 11, wherein the providing step further includes overmolding at least one of the first and second fabric ring assembly onto the fabric layer.

13. The method of forming a composite seal assembly for use in a surgical access device according to any one of claims 9 to 12, wherein the securing step includes removing excess fabric from the orifice.

14. The method of forming a composite seal assembly for use in a surgical access device according to any one of claims 9 to 13, wherein the positioning step includes pressing the fabric ring assemblies into recesses of a mold.

15. The method of forming a composite seal assembly for use in a surgical access device according to any one of claims 9 to 14, wherein the approximating step includes pinching opposing central portions of each fabric layer with mating core pins.

16. The method of forming a composite seal assembly for use in a surgical access device according to any one of claims 9 to 15, wherein the forming step includes removing a thin layer of fabric at the central portion of the seal assembly to uncover the surface of the orifice such that the orifice accepts surgical instruments therethrough.

17. The surgical device according to any one of claims 1 to 8 wherein the seal member further includes a coating to reduce frictional forces on the surgical instrument or the method of forming a composite seal assembly for use in a surgical access device according to any one of claims 9 to 16, further comprising the step of applying a coating to the seal member to reduce frictional force with the surgical instruments.

18. The surgical device or the method of forming a composite seal assembly for use in a surgical access device according to claim 17, wherein the coating is selected from the group consisting of an amorphous diamond coating, a coating having ion implantation, silicon coating, hydrogel coating or TEFLON® coating.
